# EUROPEAN PATENT APPLICATION

(11) **EP 0 824 103 A1**
(43) Date of publication of application: **18.02.1998**
(21) Application number: 96112976.4
(22) Date of filing: 13.08.1996
(51) Int. Cl.: C07K 14/655, C07K 1/04

(54) **Method for synthesis of Tyr-3-octreotide**

(71) Applicant: Su, Chang-Shinn, Tau Yen (TW); Lee, Te-Wei, Ney Hu, Taipei (TW); Chang, Shiang-Rong, Chung Li, Tau Yen (TW); Chyi, Shyh-Yi, Lungtan, Tau Yen (TW); Shen, Lie-Hang, Chung Li, Tau Yen (TW); Tsai, Zei-Tsan, Chung Li, Tau Yen (TW)
(72) Inventor: Su, Chang-Shinn, Tau Yen (TW); Lee, Te-Wei, Ney Hu, Taipei (TW); Chang, Shiang-Rong, Chung Li, Tau Yen (TW); Chyi, Shyh-Yi, Lungtan, Tau Yen (TW); Shen, Lie-Hang, Chung Li, Tau Yen (TW); Tsai, Zei-Tsan, Chung Li, Tau Yen (TW)
(74) Representative: Winkler, Andreas, Dr.

(57) **Abstract**

The present invention is a novel synthesis of Tyr-3-octreotide for radiopharmaceuticals. In this method, the starting material, Fmoc-Cys(Trt)-Wang Resin, is coupled with various selected amino acids to form a straight chain of peptide-resin compound, D-Phe-Cys(Trt)-Tyr(tBu)-D-Trp(Boc)-Lys(Boc)-Thr(tBu)-Cys(Trt)-Wang Resin. This compound then reacts with iodine to give disulfide-containing peptide resin of 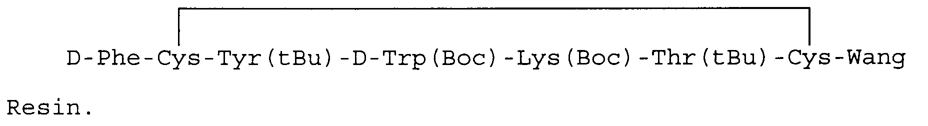
D-Phe-Cys-Tyr(tBu)-D-Trp(Boc)-Lys(Boc)-Thr(tBu)-Cys-Wang
Resin.
 Cleavage of the peptide from the resin is achieved by aminolysis with threoninol, the cleavaged peptide is further deprotected with trifluoroacetic acid (TFA) to obtain the crude peptide Tyr-3-octreotide of the formula D-Phe-Cys-Tyr-D-Trp-Lys-Thr-Cys-Thr(ol) which can be purified by high performance liquid chromatography (HPLC). The final product Tyr-3-octreotide can be labelled by radioiodine for tumor imaging radiopharmaceuticals.

## Description

### FIELD OF THE INVENTION

The present invention relates to a novel synthesis of Tyr-3-octreotide by solid phase synthesis. In particular, the present invention relates to cycliation, aminolysis, and radioactive isotope labelling such as by I-123.

### BACKGROUND OF THE INVENTION

Radiolabelled somatostatin analogue, ¹²³I-Tyr-3-octreotide, this new scintigraphic technique has attracted a great interest in clinical nuclear medicine. This pharmaceutical has been used successfully for the localization of primary and metastatic somatostatin receptor-rich tumors, such as carcinoids, islet cell tumors of the pancreas, paragangliomas and small-cell carcinomas of the lungs. Octreotide comprises 8 amino acids which has the following structural formula: 
D-Phe-Cys-Phe-D-Trp-Lys-Thr-Cys-Thr(ol)

1 2 3 4 5 6 7 8
 wherein the sulfur atoms of the Cys at the position 2 and of the Cys at the position 7 are mono-cyclic to form an -S-S- bridge. In Tyr-3-octreotide, an amino acid Tyr replaces Phe at the position 3 to make radioiodination possible. The synthetic process of octreotide has been described by Bauer et al. in U.S. Pat. 4,395,403 in 1983. The process comprises: (i) removing protected group from peptide; (ii) linking together by an amide bond two peptide units; (iii) converting a function group at the N- or C-terminal; (iv) oxidizing a straight chain polypeptide by boron-tris-trifluoroacetate. This process involves a time-consuming, multi-step synthesis, and it is difficult to separate Tyr-3-octreotide from the reaction mixtures since all the synthesis steps are carried out in liquid phase. The present invention provides a solid-phase synthesis of Tyr-3-octreotide using Fmoc methodology.

### SUMMARY OF THE INVENTION

The main object of the present invention is to provide a new method for synthesis of Tyr-3-octreotide. Tyr-3-octreotide has been synthesized using Fmoc method of solid-phase synthesis. The D-Phe-Cys(Trt)-Tyr(tBu)-D-Trp(Boc)-Lys(Boc)-Thr(tBu)-Cys(Trt)-Resin was suspended in DMF and treated with iodine. Disulfide-containing peptide-resin of D-Phe-Cys-Tyr(tBu)-D-Trp(Boc)-Lys(Boc)-Thr(tBu)-Cys-Resin can be obtained when oxidation is carried out on peptide chain anchored to polymeric supports. Cleavage of the peptide from the peptide-resin was achieved by aminolysis with threoninol. The filtrate of D-Phe-Cys-Tyr(tBu)-D-Trp(Boc)-Lys(Boc)-Thr(tBu)-Cys-Thr(ol) from the resin was deprotected with trifluoroacetic acid (TFA). Purification of the crude peptide of D-Phe-Cys-Tyr-D-Trp-Lys-Thr-Cys-Thr(ol)    (Tyr-3-octreotide) has been accomplished in a one-step procedure using reverse-phase high performance liquid chromatography (HPLC). This novel method for synthesis of Tyr-3-octreotide has been proved to be more time-saving and easier for separation of Tyr-3-octreotide from the reaction system than the prior art is.

### DETAILED DESCRIPTION OF THE INVENTION

The following abbreviations are employed:
Fmoc: 9-fluorenylmethoxycarbonyl
HAS: human serum albumin
Boc: t-Butyloxycarbonyl
tBu: tert-Butyl
Trt: triphenylmethyl
Thr(ol): the threoninol residue
Phe: the phenylalanine residue
Cys: the cysteine residue
Tyr: the tyrosine residue
Thr: the threonine residue
Lys: the lysine residue
Trp: the tryptophan residue
TFA: trifluoroacetic acid
EDT: 1,2-ethanedithiol
Wang Resin: 4-alkoxybenzyl alcohol resin
HBTU: [2-(1H-benzotriazol-1-yl)-1,1,3,3-tetramethyluronium hexafluorophosphate]

The practice of the invention is further illustrated by the following examples.

### Example 1

### Synthesis of D-Phe-Cys(Trt)-Tyr(tBu)-D-Trp(Boc)-Lys(Boc)-Thr(tBu)-Cys(Trt)-Wang Resin

Fmoc-Cys(Trt)-Wang Resin (1 mmole) was used as the staring material for solid-phase peptide synthesis. The Fmoc protecting group of Fmoc-Cys(Trt)-Wang Resin was removed by piperidine. Fmoc-Thr(tBu)-OH was activated by using HBTU. In the coupling step, the activated Fmoc-Thr(tBu)-OH reacted with Cys(Trt)-Wang Resin to form Fmoc-Thr(tBu)-Cys(Trt)-Wang Resin. Deprotecting and coupling steps are repeated with each subsequent amino acid until an assembly chain D-Phe-Cys(Trt)-Tyr(tBu)-D-Trp(Boc)-Lys(Boc)-Thr(tBu)-Cys(Trt)-Wang Resin has been completed. The peptide resin was further treated with 2.5%EDT-2.5%H₂O-95%TFA for 3 hr to form a compound giving a major peak on analytical reversed-phase high performance liquid chromatography. Positive ion electrospray mass spectra (ESMS) analysis of the isolated peak suggests the compound has a structure D-Phe-Cys-Tyr-D-Trp-Lys-Thr-Cys, as [M+H]⁺=950 Da.

### Example 2

### Synthesis of

D-Phe-Cys(Trt)-Tyr(tBu)-D-Trp(Boc)-Lys(Boc)-Thr(tBu)-

Cys(Trt)-Wang Resin.

D-Phe-Cys(Trt)-Tyr(tBu)-D-Trp(Boc)-Lys(Boc)-Thr(tBu)-Cys(Trt)-Wang Resin (50 mg) was suspended in DMF. This solution of peptide-resin was added by dropwise to a vigorously stirred solution of iodine (35.7g) in DMF. After a reaction time of 1 hr, excess iodine was washed with DMF. The product of D-Phe-Cys-Tyr-D-Trp-Lys-Thr-Cys was reductively cleavaged as described in Example 1. The analytic result gave [M+H]⁺=948 Da by ESMS.

### Example 3

### Preparation of Threoninol

Threoninol(Bzl)HCl (2 g) was dissolved in 4 ml H₂O and NaOH (0.0088 mole) was added to the solution by dropwise. The solution was extracted with 40 ml of ether for four times. After drying, the ether was removed. The residue was added to 10% Pd/c (300 mg) under hydrogen for 24 hours. When the reaction was complete, the mixture was filtered and concentrated in vacuo to a pale yellow oil. Threoninol was loaded onto silicon gel and eluted with chloroform/methanol/25% ammonium water (4/1/0.1). Thin layer chromatography (TLC) determined Thr(ol) (Rf: 0.1) and Thr(ol)Bzl (Rf: 0.68).

### Example 4

Synthesis of D-Phe-Cys-Tyr-D-Trp-Lys-Thr-Cys-Thr(ol)

D-Phe-Cys-Tyr(tBu)-D-Trp(Boc)-Lys(Boc)-Thr(tBu)-Cys-

Wang Resin (300 mg) and Thr(ol) (674 mg)

were stirred in 3 ml of DMF for 18 hours at room temperature. After removal of the precipitated resin by filtration, the filtrate was evaporated under reduced pressure. 10 ml water was added to the residue and the precipitate was filtered off. The precipitate was treated with 3 ml of 95% TFA for 1.5 hours, diluted with 3 ml of water, concentrated to about 1 ml in vacuo. The mixture was added with cold ether and the precipitate was filtered off and washed with ether. The crude product was purified by reverse-phase HPLC. The product gave [M+H]⁺=1035 Da by ESMS.

### Example 5

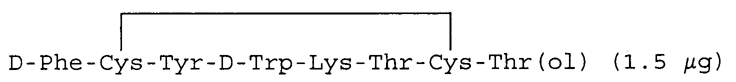

Preparation of D-Phe-Cys-Tyr-D-Trp-Lys-Thr-Cys-Thr(ol)
123I

was dissolved in 0.05 M acetic acid. To the solution in a 10 ml vial was added successively 20 µl 0.05 M phosphate (pH 7.5) and iodine-123. The mixture was stirred for 3 min at room temperature, then 1.7 µg chloramine-T (in 25 µl 0.05 M phosphate) was added. The solution was vortexed for 1 min. The iodination was stopped by the addition of 0.1 ml 10% human serum albumin. After vortexing for 30 seconds, 1.85 ml 5 mM ammonium acetate was added. Purification was performed by using a SEP-PAK C18 reversed-phase extraction cartridge which was prewashed with 5 ml 70% ethanol and subsequently activated with 5 ml 2-propanol. After loading the sample, the cartridge was washed successively with 5 ml water and 5 ml 0.5 M acetic acid. Radioiodinated product was eluted with 5 ml 96% ethanol. Quality analysis was done on ITLC (SG) with 85% methanol to determine I-123 (Rf=1) and I-123-Tyr-3-octreotide (Rf=0).

## Claims

1. A method for synthesis of Tyr-3-octreotide using Fmoc-Cys(Trt)-Wang Resin as the starting material, comprising the steps of coupling of various selected amino acid residues to give a straight chain of peptide-resin compound, oxidizing this compound, then cleavaging the oxidized peptide of a mono-cyclic chain from the resin to the liquid phase, and deprotecting the cleavaged peptide to obtain Tyr-3-octreotide of formula

D-Phe-Cys-Tyr-D-Trp-Lys-Thr-Cys-Thr(ol)

2. The method for synthesis of Tyr-3-octreotide according to claim 1, wherein said straight chain of peptide-resin compound has the formula D-Phe-Cys(Trt)-Tyr(tBu)-D-Trp(Boc)-Lys(Boc)-Thr(tBu)-Cys(Trt)-Wang Resin.

3. The method for synthesis of Tyr-3-octreotide according to claim 1, wherein said oxidizing reaction which comprises the steps of reacting peptide-resin compound with iodine to form a mono-cyclic chain of peptide of formula

D-Phe-Cys-Tyr(tBu)-D-Trp(Boc)-Lys(Boc)-Thr(tBu)-Cys-Wang Re-
sin.

4. The method for synthesis of Tyr-3-octreotide according to claim 1, wherein said cleavaging reaction which comprises the steps of aminolyzing the oxidized peptide of a mono-cyclic chain with threoninol to give octreotide of formula
